# EUROPEAN PATENT APPLICATION

(11) **EP 3 438 874 A1**
(43) Date of publication of application: **06.02.2019**
(21) Application number: 18176789.8
(22) Date of filing: 08.06.2018
(51) Int. Cl.: G06K 9/00

(54) **IMAGE ANALYSIS SYSTEM COMPRISING A GATING UNIT FOR DELIMITING AN ANALYSIS OBJECT**

(30) Priority: 31.07.2017 JP 2017147381
(71) Applicant: OLYMPUS CORPORATION, Hachioji-shi Tokyo 192-8507 (JP)
(72) Inventor: MORI, Keigo, Tokyo 192-8507 (JP)
(74) Representative: Gunzelmann, Rainer

(57) **Abstract**

The present invention makes a delimiting operation easy and reduces unintended delimitation due to an erroneous operation. An image analysis system 1 includes: an analysis-object recognition unit 11 for recognizing analysis objects from images; a graph generation unit 13 for generating a graph on the basis of at least one measurement parameter of the analysis objects recognized by the analysis-object recognition unit 11; a list generation unit 15 for generating a list of the images of the analysis objects recognized by the analysis-object recognition unit 11; a gating unit 17 that generates, on the graph, a partial region for delimiting an analysis object selected by the user on the graph or in the list and that edits the partial region so as to include in the partial region, or exclude from the partial region, the analysis object selected by the user on the graph or in the list.

## Description

### {Technical Field}

The present invention relates to an image analysis system.

### {Background Art}

There is a well-known image analysis system for recognizing an analysis object, such as a cell, in an image and analyzing the recognized analysis object (refer to, for example, Patent Literature 1). In the image analysis system described in Patent Literature 1, a partial region including cells, which are analysis objects, is generated and edited in a histogram generated on the basis of a certain measurement parameter, thus narrowing and delimiting (gating) the analysis objects. Thereafter, image data of each cell included in the partial region is displayed in the form of a gallery, it is determined whether or not delimitation is appropriate for each cell, and the partial region in the histogram is finely corrected so as to exclude, from the partial region, cells determined as being inappropriately delimited.

### {Citation List}

### {Patent Literature}

{PTL 1}
Japanese Unexamined Patent Application, Publication No. 2010-151677

### {Summary of Invention}

### {Technical Problem}

However, in the image analysis system described in Patent Literature 1, it is necessary to repeat the operation of confirming each of the delimited cells in the gallery and finely correcting the partial region in the histogram. This operation is complicated and time-consuming, possibly leading to incorrect delimitation due to an erroneous operation.

The present invention has been conceived in light of the above-described circumstances, and an object thereof is to provide an image analysis system that makes the delimiting operation easy and allows a reduction in unintended delimitation due to an erroneous operation.

### {Solution to Problem}

In order to achieve the above-described object, the present invention provides the following solutions.

One aspect of the present invention is an image analysis system including: an analysis-object recognition unit for recognizing analysis objects from images; a graph generation unit for generating a graph on the basis of at least one measurement parameter of the analysis objects that have been recognized by the analysis-object recognition unit; a list generation unit for generating a list of the images of the analysis objects that have been recognized by the analysis-object recognition unit; and a gating unit that generates, on the graph, a partial region for delimiting an analysis object selected by a user on the graph or in the list and that edits the partial region so as to include in the partial region, or exclude from the partial region, the analysis object selected by the user on the graph or in the list.

According to this aspect, when the analysis objects are recognized from the images by the analysis-object recognition unit, the graph generation unit generates a graph of the recognized analysis objects, and the list generation unit generates a list of the images of the recognized analysis objects. In addition, when the user selects an analysis object on the graph or in the list, the gating unit generates, on the graph, a partial region for delimiting the selected analysis object. Thereafter, when the user selects, on the graph or in the list, an analysis object to be included in the partial region, the gating unit edits the partial region so as to include the selected analysis object, and when the user selects an analysis object to be excluded from the partial region, the gating unit edits the partial region so as to exclude the selected analysis object.

Therefore, the user can not only edit the partial region on the graph but also correct the partial region on the graph from an image in the list. Therefore, the user can be relieved from repeating the operation of checking each delimited analysis object in the list and then finely correcting the partial region on the graph. In addition, the user can intuitively determine the validity of delimitation from an image in the list. Therefore, the delimiting operation can be made easy, and a desired analysis object can be delimited with high accuracy.

In the above-described aspect, the gating unit may generate the partial region as a certain range on the graph, the certain range being centered on the analysis object selected by the user in the list.

With this structure, merely by selecting a typical analysis object from the image in the list, a partial region including an analysis object having a similar measurement parameter can be easily generated.

In the above-described aspect, the gating unit may generate the partial region as a range formed by enlarging, by a certain amount, a minimum range, on the graph, including a plurality of the analysis objects selected by the user in the list.

With this structure, merely by selecting a plurality of typical analysis objects from images, a partial region including analysis objects having a similar measurement parameter can be easily generated.

In the above-described aspect, the gating unit may generate the partial region as a range formed by reducing, by a certain amount, a maximum range, on the graph, not including a plurality of the analysis objects selected by the user in the list.

With this structure, merely by selecting a plurality of undesired analysis objects from images, a partial region not including undesired analysis objects can be easily generated.

In the above-described aspect, the gating unit may generate the partial region that includes the analysis object specified by the user in the list as to be included in the partial region and that excludes the analysis object specified as to be excluded from the partial region.

With this structure, a partial region including a desired analysis object and excluding an undesired analysis object can be generated easily and precisely.

In the above-described aspect, the gating unit may edit the partial region so as to exclude the analysis object specified by the user in the list as to be excluded from the partial region and so as to include the analysis object specified by the user in the list as to be included in the partial region.

With this structure, the partial region can be easily corrected to a precise partial region including a desired analysis object and not including an undesired analysis object.

### {Advantageous Effects of Invention}

The present invention affords an advantage in that the delimiting operation is made easy and unintended delimitation due to an erroneous operation can be reduced.

### {Brief Description of Drawings}

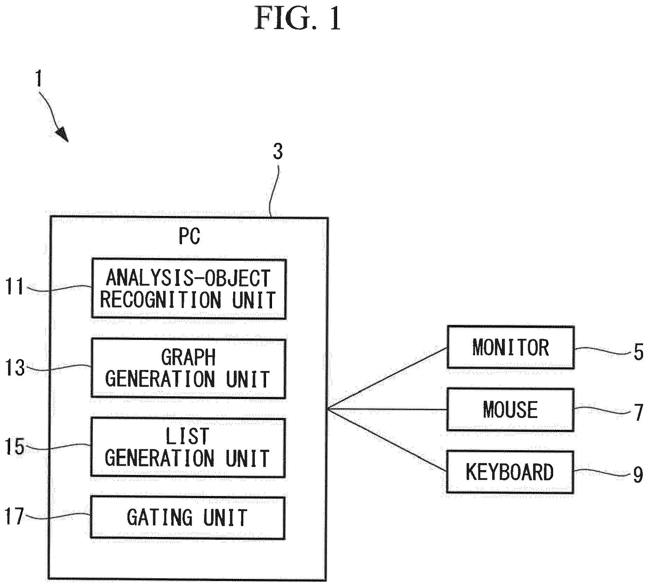

{Fig. 1}
   Fig. 1 is a schematic configuration diagram showing an image analysis system according to an embodiment of the present invention.
{Fig. 2}
   Fig. 2 is a diagram showing one example of a scatter diagram generated by a graph generation unit of the image analysis system in Fig. 1.
{Fig. 3}
   Fig. 3 is a diagram showing one example of a list of cells generated by a list generation unit of the image analysis system in Fig. 1.
{Fig. 4}
   Fig. 4 is a diagram showing one example of a partial region displayed on a scatter diagram by the image analysis system in Fig. 1.
{Fig. 5}
   Fig. 5 is a diagram showing one example of a dialog for generating a partial region displayed on a monitor in the image analysis system in Fig. 1.
{Fig. 6}
   Fig. 6 is a diagram showing one example of a partial region displayed in a histogram in an image analysis system according to a modification of the embodiment of the present invention.
{Fig. 7}
   Fig. 7 is a diagram showing one example of a dialog for generating a partial region displayed on a monitor in an image analysis system according to a first modification of the embodiment of the present invention.
{Fig. 8}
   Fig. 8 is a diagram showing one example of a partial region displayed in a histogram in the image analysis system according to the first modification of the embodiment of the present invention.
{Fig. 9}
   Fig. 9 is a diagram showing one example of a partial region displayed on a scatter diagram in the image analysis system according to the first modification of the embodiment of the present invention.
{Fig. 10}
   Fig. 10 is a diagram showing one example of a dialog for generating a partial region displayed on a monitor in an image analysis system according to a second modification of the embodiment of the present invention.
{Fig. 11}
   Fig. 11 is a diagram showing one example of a partial region displayed in a histogram in the image analysis system according to the second modification of the embodiment of the present invention.
{Fig. 12}
   Fig. 12 is a diagram showing one example of a partial region displayed on a scatter diagram in the image analysis system according to the second modification of the embodiment of the present invention.
{Fig. 13}
   Fig. 13 is a diagram showing one example of a menu for allowing a user to select whether a selected cell is to be included in or excluded from a partial region in an image analysis system according to a third modification of the embodiment of the present invention.
{Fig. 14}
   Fig. 14 is a diagram showing one example of a list of cells generated by a list generation unit in the image analysis system according to the third modification of the embodiment of the present invention.
{Fig. 15}
   Fig. 15 is a diagram showing one example of a dialog for generating a partial region displayed on a monitor in the image analysis system according to the third modification of the embodiment of the present invention.
{Fig. 16}
   Fig. 16 is a diagram showing one example of a partial region displayed in a histogram in the image analysis system according to the third modification of the embodiment of the present invention.
{Fig. 17}
   Fig. 17 is a diagram showing one example of a partial region displayed on a scatter diagram in the image analysis system according to the third modification of the embodiment of the present invention.
{Fig. 18}
   Fig. 18 is a diagram showing another example of a partial region displayed on a scatter diagram in the image analysis system according to the third modification of the embodiment of the present invention.
{Fig. 19}
   Fig. 19 is a diagram showing still another example of a partial region displayed on a scatter diagram in the image analysis system according to the third modification of the embodiment of the present invention.
{Fig. 20}
   Fig. 20 is a diagram showing one example of a menu for allowing a user to instruct the correction of a partial region in an image analysis system according to a fourth modification of the embodiment of the present invention.
{Fig. 21}
   Fig. 21 is a diagram showing one example of a partial region displayed in a histogram in the image analysis system according to the fourth modification of the embodiment of the present invention.
{Fig. 22}
   Fig. 22 is a diagram showing one example of a partial region displayed on a scatter diagram in the image analysis system according to the fourth modification of the embodiment of the present invention.
{Fig. 23}
   Fig. 23 is a diagram for illustrating the configuration of a PC of an image analysis system according to a fifth modification of the embodiment of the present invention.

### {Description of Embodiments}

An image analysis system according to one embodiment of the present invention will now be described with reference to the drawings.

As shown in Fig. 1, an image analysis system 1 according to this embodiment includes: a personal computer (PC) 3 for processing images; a monitor 5 for displaying images, information, and so forth; and a mouse 7 and a keyboard 9 for allowing a user to enter an instruction.

The PC 3 includes: an analysis-object recognition unit 11 for recognizing an analysis object from an image; a graph generation unit 13 for generating a graph on the basis of at least one measurement parameter of analysis objects that have been recognized by the analysis-object recognition unit 11; a list generation unit 15 for generating a list of images of the analysis objects that have been recognized by the analysis-object recognition unit 11; and a gating unit 17 for drawing and editing, on the graph, a partial region for delimiting analysis objects that have been selected by the user. The analysis-object recognition unit 11, graph generation unit 13, list generation unit 15, and gating unit 17 are installed in the PC 3 in the form of software.

The analysis-object recognition unit 11 recognizes a cell, a spheroid, a cell organ, and so forth as the analysis object on the basis of image information that has been read into the PC 3. This embodiment will be described by way of an example of a cell as the analysis object.

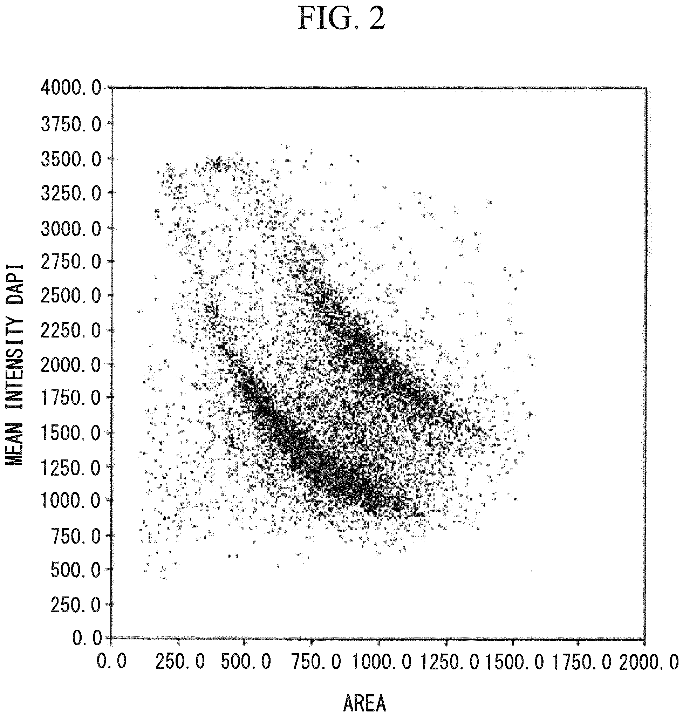

The graph generation unit 13 measures, as measurement items, the area, dye-by-dye luminance value, and so forth of cells recognized by the analysis-object recognition unit 11. The graph generation unit 13 draws a graph, such as a histogram and a scatter diagram, on the basis of at least one measurement parameter that has been obtained by the measurement and displays the graph on the monitor 5. Fig. 2 shows one example of a scatter diagram serving as the graph. In Fig. 2, the vertical axis represents the luminance value of DAPI (4',6-diamidino-2-phenylindole), which is a type of fluorescent dye, the horizontal axis represents the area of the cell, and each dot represents a cell.

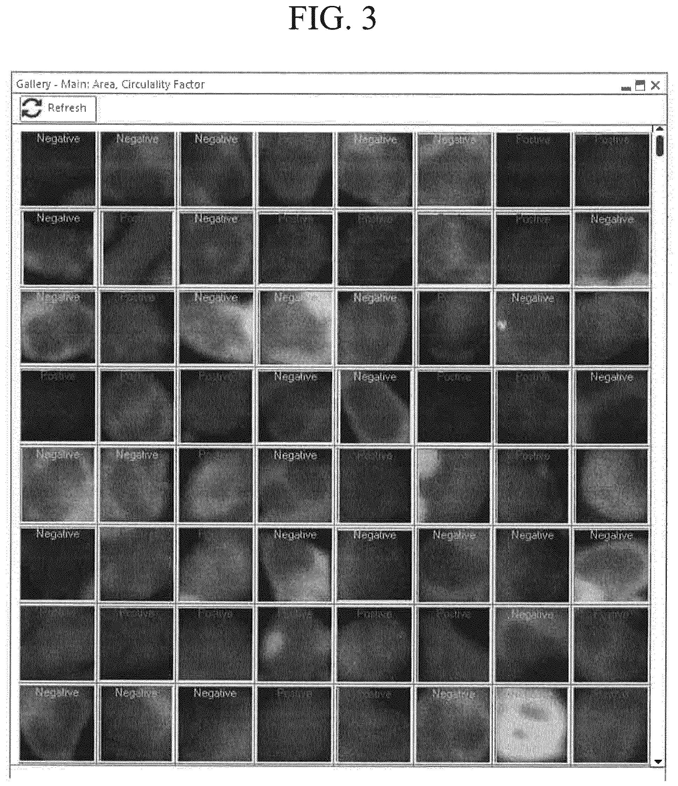

As shown in Fig. 3, the list generation unit 15 generates a list (gallery) of thumbnail images of the cells recognized by the analysis-object recognition unit 11 and displays the list on the monitor 5. This list generation unit 15 generates and displays a list of all the recognized cells or, alternatively, generates a list of cells that are included, as well as a list of cells that are not included, in a partial region delimited by the gating unit 17 and switches between these two types of list for display. In Fig. 3, the images constituting the list are shown as thumbnail images of cells.

When the user selects cells on the graph generated by the graph generation unit 13 or in the list generated by the list generation unit 15, the gating unit 17 draws, on the graph, a partial region for delimiting the selected cells. The gating unit 17 also edits the partial region on the graph so as to include, in the partial region, the cells selected by the user on the graph or in the list or exclude such cells from the partial region.

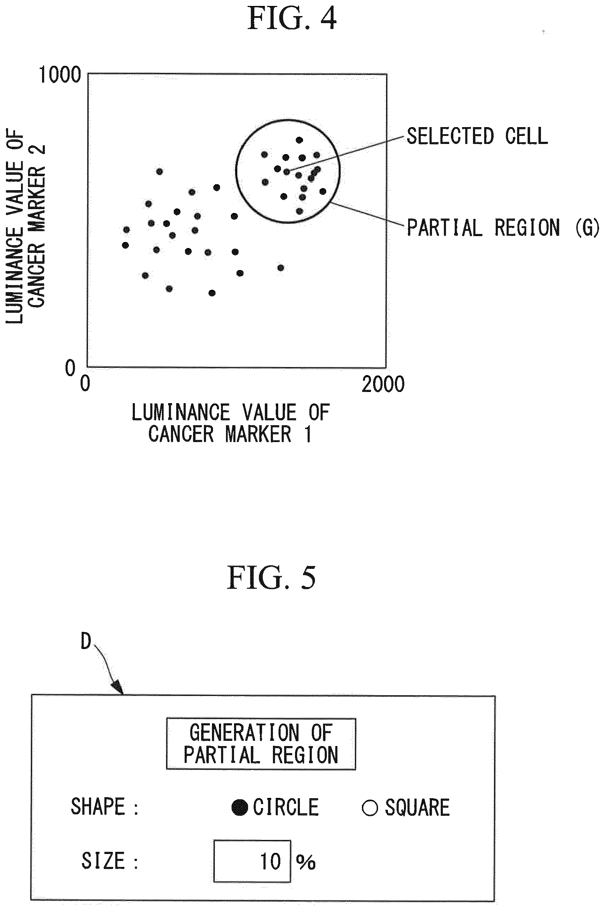

More specifically, as shown in Fig. 4, the gating unit 17 draws a partial region G, which is circular, rectangular, or the like, indicating a certain range on the graph, the range being centered on the cell selected by the user in the list, and displays the partial region G on the graph on the monitor 5. In Fig. 4, the horizontal axis represents the luminance value of a cancer marker 1, and the vertical axis represents the luminance value of a cancer marker 2, showing a circular partial region G as an example.

In addition, the gating unit 17 edits the partial region G on the graph so as to exclude from the partial region G the cell that has been specified by the user in the list as to be excluded and also edits the partial region G on the graph so as to include in the partial region G the cell that has been specified by the user in the list as to be included.

The operation of the image analysis system 1 with this structure will be described.

In order to analyze images with the image analysis system 1 according to this embodiment, the user first instructs the software in the PC 3, by using the mouse 7 and the keyboard 9, to read images of cells serving as analysis objects.

When the images of the cells are read into the PC 3, the user instructs the software in the PC 3, by using the mouse 7 and the keyboard 9, to recognize the cells serving as the analysis objects and to measure the recognized cells. In the PC 3, on the basis of the user's instruction, the analysis-object recognition unit 11 recognizes the cells serving as the analysis objects on the basis of the read image information. Thereafter, the graph generation unit 13 measures the areas and the dye-by-dye luminance values of the cells recognized by the analysis-object recognition unit 11 and, on the basis of these measurement parameters, generates a scatter diagram (graph) as shown in Fig. 2 and displays the diagram on the monitor 5.

Next, the user instructs the software in the PC 3, by using the mouse 7 and the keyboard 9, to generate a list of the images of the cells recognized by the analysis-object recognition unit 11. In the PC 3, on the basis of the user's instruction, the list generation unit 15 collects thumbnail images of the recognized cells and then generates a list as shown in Fig. 3 and displays the list on the monitor 5. In this case, because no partial regions have been generated yet, a list of all the recognized cells is generated.

Next, the user selects the cell of interest by right-clicking the mouse 7 on the thumbnail image of a desired cell in the list displayed on the monitor 5. To measure, for example, the number of cancer cells, the user selects a typical cell glowing with a cancer marker.

Next, the user instructs the software in the PC 3, by using the mouse 7 and the keyboard 9, to draw the partial region G. In the PC 3, on the basis of the user's instruction, the gating unit 17 generates a circular partial region G, as shown in Fig. 4, indicating a certain range on the scatter diagram, the range being centered on the selected cell, and then the generated partial region G is displayed on the scatter diagram on the monitor 5.

The size of the partial region G may be edited on the scatter diagram by the user specifying a numerical value. For example, a dialog D for generating a partial region may be displayed on the monitor 5, as shown in Fig. 5, so that the user is allowed to select the shape and the size (range) of the partial region G. The user may be allowed to set the size of the partial region G by entering, for example, the diameter or the length of one side as a percentage of the minimum to maximum value of the area of the scatter diagram.

Subsequently, in a case where there is a cell that the user wants to include in the partial region G or a cell that the user wants to exclude from the partial region G, the user selects the relevant cell on the scatter diagram or in the list displayed on the monitor 5 and instructs the software in the PC 3, by using the mouse 7 and the keyboard 9, to perform editing for including the relevant cell in the partial region G or excluding the relevant cell from the partial region G.

In the PC 3, when editing for including the cell in the partial region G is instructed, the gating unit 17 edits the partial region G on the scatter diagram so as to include the selected cell in the partial region G. In addition, when editing for excluding the cell from the partial region G is instructed, the gating unit 17 edits the partial region G on the scatter diagram so as to exclude the selected cell from the partial region G.

Subsequently, once the partial region G for the cell of interest is drawn, the user applies the intended measurement and analysis to the cells included in that partial region G. For example, the user measures the number of cells or the shapes of the cells included in the partial region G and carries out statistical analysis.

As described above, according to the image analysis system 1 of this embodiment, the gating unit 17 allows the user not only to edit the partial region G on the scatter diagram but also to correct the partial region G on the scatter diagram from an image in the list, thus relieving the user from having to repeat the operation of checking each of the delimited cells in the list and then finely correcting the partial region G on the scatter diagram. Furthermore, it is possible to intuitively determine the validity of delimitation from images in the list. Therefore, the delimiting operation can be simplified, thereby making it possible to delimit the desired cells with high accuracy.

In addition, the gating unit 17 sets, as the partial region G, a certain range on the scatter diagram, the range being centered on the cell selected by the user in the list displayed on the monitor 5, and thus, merely by selecting a typical cell from the images in the list, the user can easily generate the partial region G including cells having similar measurement parameters.

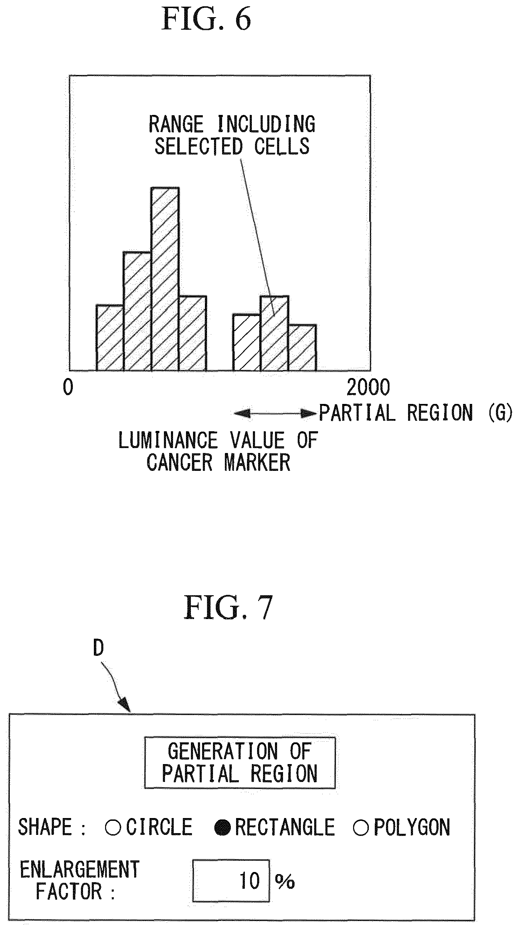

Although this embodiment has been described by way of an example of a scatter diagram serving as a graph, the graph generation unit 13 may generate, for example, a histogram as shown in Fig. 6. In this case, the gating unit 17 may display, as the partial region G in a histogram, a certain range (±N) including the cell selected by the user. In Fig. 6, the horizontal axis represents the luminance value of a cancer marker.

This embodiment can be modified as follows.

A first modification may be implemented such that, for example, the gating unit 17 sets, as the partial region G, a range formed by enlarging, by a certain amount, the minimum range on a graph including a plurality of cells selected by the user in the list of cells.

In this case, the user may select a plurality of cells of interest by left-clicking the mouse 7 while holding the Ctrl key of the keyboard 9 in the list of cells displayed on the monitor 5 and may confirm the selection by right-clicking the mouse 7 when the selection is completed. For example, when the shape of the cell of interest is to be measured, the user may select a plurality of cells glowing with the marker dying the cell of interest.

In this case, for example, as shown in Fig. 7, the dialog D for generating a partial region may be displayed on the monitor 5, so that the user is allowed to select the shape and the size (range) of the partial region G. The size of the partial region G may be set by entering a percentage by which the minimum range including the plurality of selected cells is enlarged.

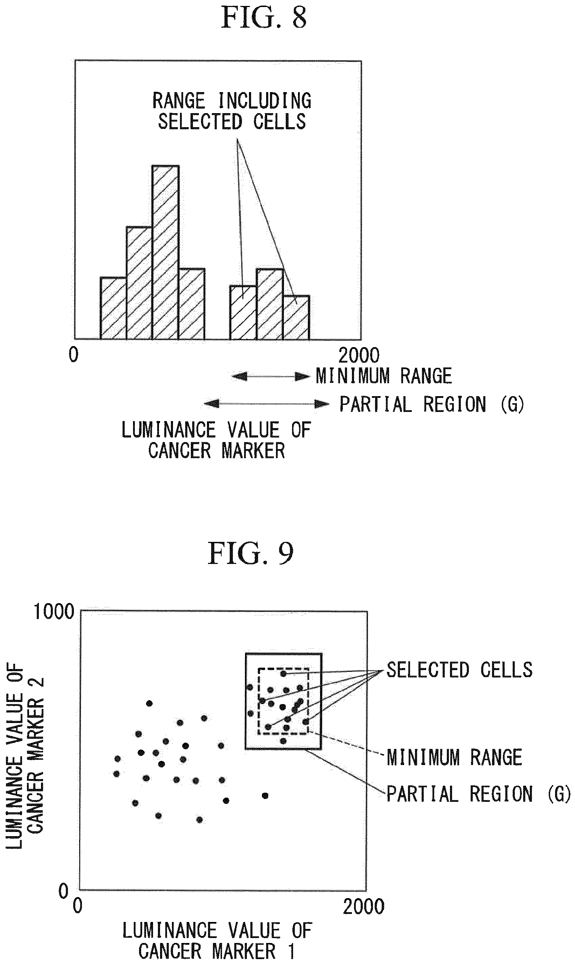

In the case of a histogram, for example, as shown in Fig. 8, it is advisable that the gating unit 17 generate, as the partial region G, a range (X to X+N) formed by enlarging, by a certain size, the minimum range including the selected cells and then display the partial region G. In addition, in the case of a scatter diagram, for example, as shown in Fig. 9, it is advisable that the gating unit 17 generate a circular, rectangular, or polygonal partial region G formed by enlarging, by a certain size, the minimum range including the selected cells and then display the partial region G.

According to this modification, merely by selecting a plurality of typical cells from the thumbnail images in the list, it is possible to easily generate a partial region G including cells having a similar measurement parameter and then display the partial region G in the histogram or the scatter diagram.

A second modification may be implemented such that the gating unit 17 sets, as the partial region G, a range formed by reducing, by a certain amount, the maximum range on the graph not including a plurality of cells selected by the user in the list of cells.

In this case, the user may select a plurality of cells to be excluded by left-clicking the mouse 7 while holding the Ctrl key of the keyboard 9 in the list of cells displayed on the monitor 5 and, when the selection is completed, confirm the selection by right-clicking the mouse 7. For example, in order to measure the shape of the cell of interest, the user may select a plurality of cells the shapes of which are abnormal or cells not glowing with the marker dying the cell of interest.

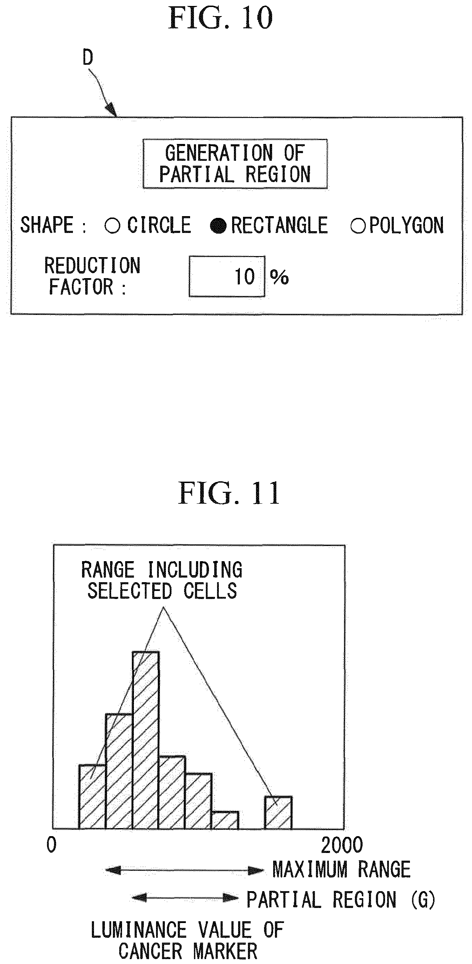

In this case, a dialog D for generating a partial region may be displayed on the monitor 5 as shown in, for example, Fig. 10 so that the user can select the shape and the size (range) of the partial region. The size of the partial region may be set, for example, by entering a percentage by which the maximum range, on the scatter diagram, not including the plurality of selected cells is reduced.

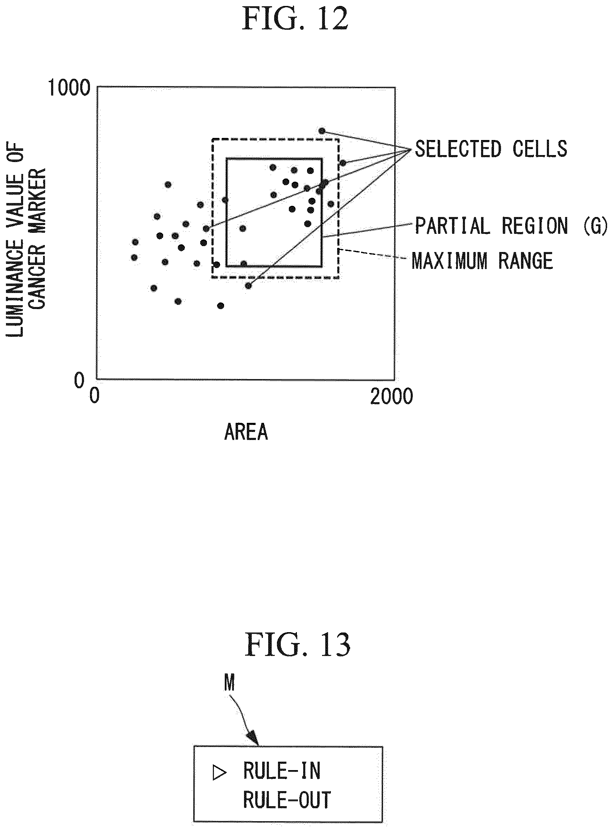

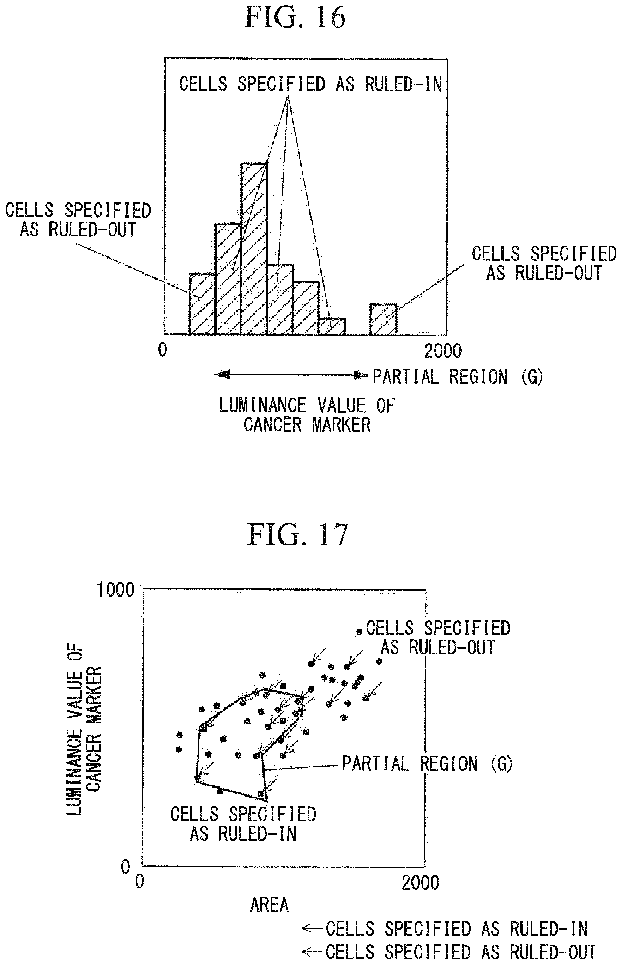

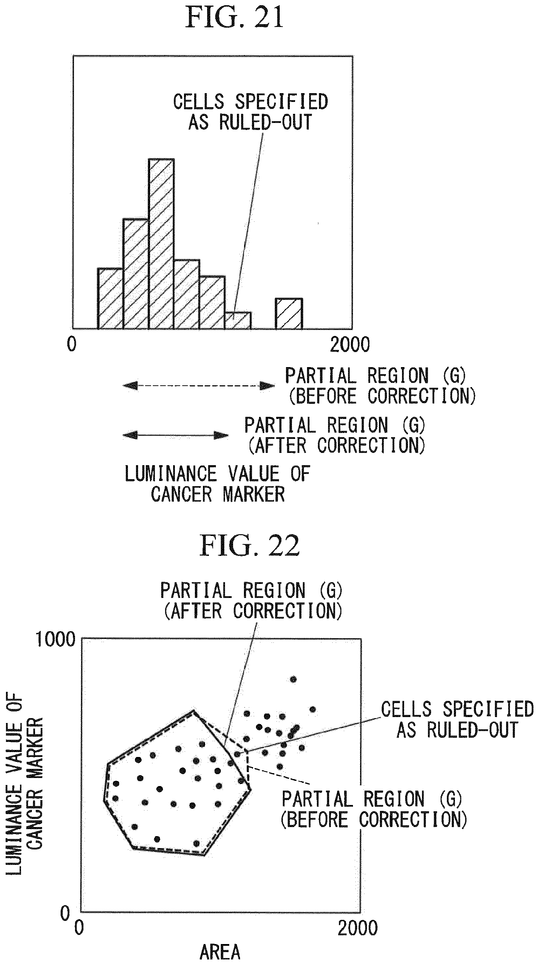

In the case of a histogram, for example, as shown in Fig. 11, it is advisable that the gating unit 17 generate, as the partial region G, a range (X to X+N) formed by reducing, by a certain size, the maximum range not including the selected cells and then display the partial region G. In addition, in the case of a scatter diagram, for example, as shown in Fig. 12, it is advisable that the gating unit 17 generate a circular, rectangular, or polygonal partial region G formed by reducing, by a certain size, the maximum range not including the selected cells and then display the partial region G. In Fig. 12, the vertical axis represents the luminance value of a cancer marker, and the horizontal axis represents the area of the cell. This also applies to Figs. 17 and 22.

According to this modification, it is possible to easily generate a partial region G not including undesired cells and to display the partial region G in the histogram or the scatter diagram merely by selecting a plurality of undesired cells from the thumbnail images in the list.

A third modification may be implemented such that the gating unit 17 generates a partial region G that includes the cells specified by the user in the list of cells as to be included in the partial region G and that does not include the cells specified by the user as to be excluded from the partial region G.

In this case, the user may select the cells of interest or the cells to be excluded by right-clicking the mouse 7 on the thumbnail images of the desired cells in the list displayed on the monitor 5. As shown in, for example, Fig. 13, a menu M ("rule in", "rule out") for allowing the user to select, for each of the selected cells, whether to rule in (include) the cell or rule out (exclude) the cell in the range of the partial region G may be displayed on the monitor 5, so that the user can select either "rule in" or "rule out" for each cell.

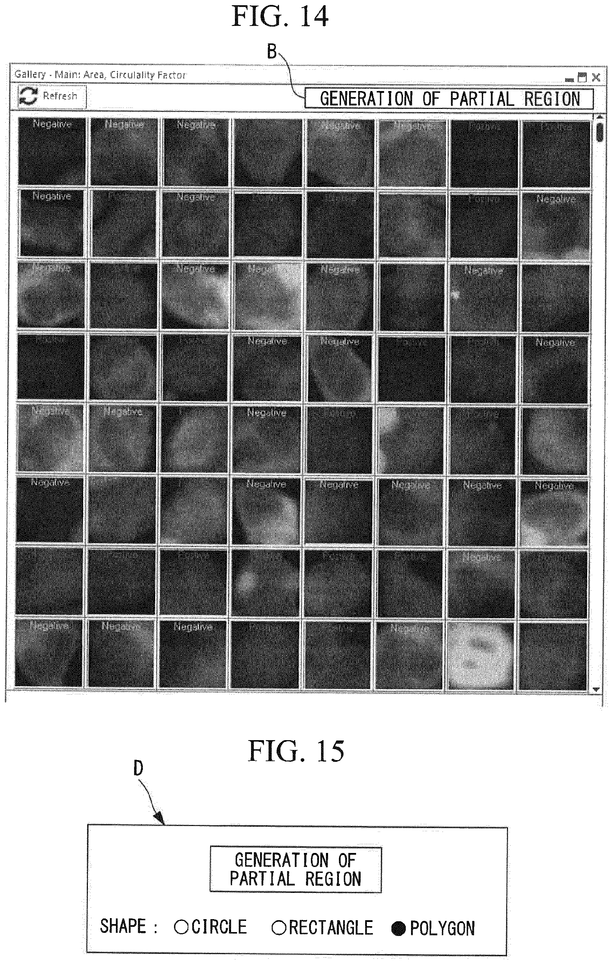

Here, for example, when the user presses a button B for generating a partial region in the list of cells displayed on the monitor 5, as shown in Fig. 14, a dialog D for generating a partial region, as shown in Fig. 15, may be displayed on the monitor 5, so that the user can select the shape of the partial region G.

In a histogram as shown in, for example, Fig. 16, the gating unit 17 may generate, as the partial region G, a range (X to X+N) that includes the cells specified as to be included in the partial region G and that does not include the cells specified as to be excluded from the partial region G and then display the partial region G. In addition, on a scatter diagram as shown in, Fig. 17, the gating unit 17 may generate a circular, rectangular, or polygonal partial region G that includes the cells specified as to be included in the partial region G and that does not include the cells specified as to be excluded from the partial region G, and then may display the partial region G.

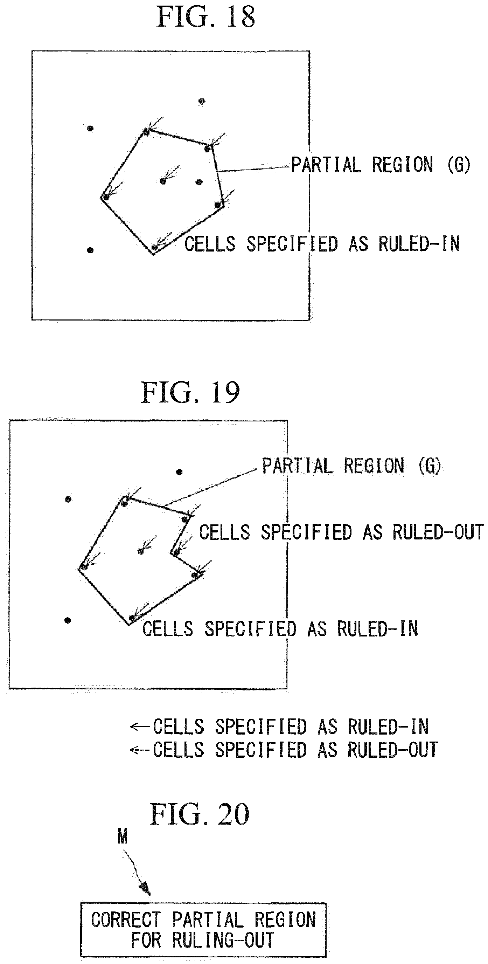

As shown in, for example, Figs. 18 and 19, the partial region G may be a polygon that includes all cells specified as to be ruled in (included) and that does not include any cells specified as to be ruled out (excluded), wherein the total length of the sides of the polygon is minimized.

According to this modification, it is possible to generate, in an easy and precise manner, the partial region G that includes desired cells and that excludes undesired cells.

A fourth modification may be implemented such that cells are narrowed by roughly pre-generating a relatively large partial region G on the graph, and subsequently, the list generation unit 15 generates a list of the cells narrowed within that partial region G. This allows the cell of interest to be easily selected in the case of a large number of cells. Thereafter, the relatively large partial region G that has been generated may be corrected so as to exclude undesired cells from the partial region G.

In this case, the user may instruct the software in the PC 3 to arrange the thumbnail images of cells in the order from the cell that is disposed farthest from the center of the partial region G. By doing so, processing for selecting the next cell is made easy.

It is advisable that the user select the cells to be excluded from the partial region G by right-clicking the mouse 7 on the thumbnail images of undesired cells in the list displayed on the monitor 5. For example, in the case where cells other than the cells of interest are included in the partial region G, those cells may be selected.

In addition, once the user selects the cells to be excluded from the partial region G, it is advisable that a menu M for correcting the partial region G from which the selected cells are excluded ("correct partial region for rule-out") be displayed on the monitor 5, as shown in, for example, Fig. 20, so that the user can instruct the correction.

The gating unit 17 may correct the partial region G so as to exclude the selected cells from the partial region G in the case of a histogram as shown in, for example, Fig. 21 and may correct the circular, rectangular, or polygonal partial region G so as to exclude the selected cells from the partial region G in the case of a scatter diagram as shown in Fig. 22.

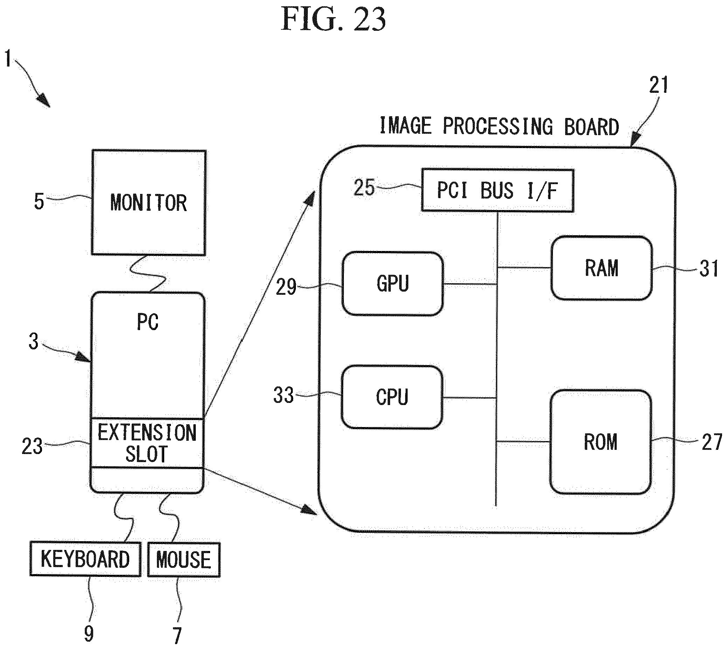

A fifth modification may be implemented such that the PC 3 executes processing with an image processing board 21, as shown in Fig. 23, instead of executing processing with the installed software.

The image processing board 21 is mounted in the PC 3 and is connected to an extension slot 23 of the PC 3 via a Peripheral Component Interconnect bus Interface (PCI BUS I/F) 25. The PCI BUS I/F 25 transmits to and receives from the PC 3 the image data of cells acquired by, for example, a microscope, not shown in the figure, and commands.

This image processing board 21 includes: a Read-Only Memory (ROM) 27; a Graphics Processing Unit (GPU) 29; a Random Access Memory (RAM) 31; and a Central Processing Unit (CPU) 33.

An analysis-object recognition program (analysis-object recognition unit), a graph generation program (graph generation unit), a list generation program (list generation unit), a gating program (gating unit), and an image-processing-board control program for operating the CPU 33 are recorded in the ROM 27.

In response to an instruction from the CPU 33, the GPU 29 executes the analysis-object recognition program and the gating program recorded in the ROM 27. This GPU 29 achieves processing similar to that of the analysis-object recognition unit 11 by executing the analysis-object recognition program and achieves processing similar to that of the gating unit 17 by executing the gating program.

The RAM 31 stores data temporarily while the GPU 29 and the CPU 33 are operating.

The CPU 33 manages the timing at which data is transmitted to and received from the PC 3 and controls the entire image processing board 21. In addition, the CPU 33 executes the graph generation program and the list generation program recorded in the ROM 27. This CPU 33 achieves processing similar to that of the graph generation unit 13 by executing the graph generation program and achieves processing similar to that of the list generation unit 15 by executing the list generation program. In addition, the CPU 33 instructs the PC 3 to transmit, to the PC 3, data resulting from executing these programs and to display the transmitted data on the monitor 5.

Dedicated hardware may be employed individually for the CPU 33 and GPU 29, or alternatively, the CPU 33 and GPU 29 may be designed so as to be embedded into one Field-Programmable Gate Array (FPGA). Furthermore, the PCI BUS I/F 25 may also be designed to be embedded into an FPGA.

Although the embodiments of the present invention have been described in detail with reference to the drawings, the specific structure is not limited to those of these embodiments but includes design changes etc. that do not depart from the spirit of the present invention. For example, the present invention is not limited to the invention applied to each of the above-described embodiments and modifications but can be applied to, for example, embodiments in which these embodiments and modifications are appropriately combined and is not particularly limited.

### {Reference Signs List}

- 1: Image analysis system
- 11: Analysis-object recognition unit
- 13: Graph generation unit
- 15: List generation unit
- 17: Gating unit
- G: Partial region

## Claims

1. An image analysis system comprising:
an analysis-object recognition unit for recognizing analysis objects from images;
a graph generation unit for generating a graph on the basis of at least one measurement parameter of the analysis objects that have been recognized by the analysis-object recognition unit;
a list generation unit for generating a list of the images of the analysis objects that have been recognized by the analysis-object recognition unit; and
a gating unit that generates, on the graph, a partial region for delimiting an analysis object selected by a user on the graph or in the list and that edits the partial region so as to include in the partial region, or exclude from the partial region, the analysis object selected by the user on the graph or in the list.

2. The image analysis system according to claim 1, wherein the gating unit generates the partial region as a certain range on the graph, the certain range being centered on the analysis object selected by the user in the list.

3. The image analysis system according to claim 1, wherein the gating unit generates the partial region as a range formed by enlarging, by a certain amount, a minimum range, on the graph, including a plurality of the analysis objects selected by the user in the list.

4. The image analysis system according to claim 1, wherein the gating unit generates the partial region as a range formed by reducing, by a certain amount, a maximum range, on the graph, not including a plurality of the analysis objects selected by the user in the list.

5. The image analysis system according to claim 1, wherein the gating unit generates the partial region that includes the analysis object specified by the user in the list as to be included in the partial region and that excludes the analysis object specified as to be excluded from the partial region.

6. The image analysis system according to one of claims 1 to 5, wherein the gating unit edits the partial region so as to exclude the analysis object specified by the user in the list as to be excluded from the partial region and so as to include the analysis object specified by the user in the list as to be included in the partial region.
